# EUROPEAN PATENT APPLICATION

(11) **EP 4 666 953 A1**
(43) Date of publication of application: **24.12.2025**
(21) Application number: 25183214.3
(22) Date of filing: 17.06.2025
(51) Int. Cl.: A61B 6/04, A61B 6/00, A61B 6/51

(54) **X-RAY IMAGING APPARATUS**

(30) Priority: 19.06.2024 KR 20240079727; 26.05.2025 KR 20250068432
(71) Applicant: Vatech Co., Ltd., Hwaseong-si, Gyeonggi-do 18449 (KR); Vatech Ewoo Holdings Co., Ltd., Hwaseong-si, Gyeonggi-do 18449 (KR)
(72) Inventor: KIM, Sung Min, 18449 Gyeonggi-do (KR)
(74) Representative: Lorenz & Kollegen

(57) **Abstract**

Proposed is an X-ray imaging apparatus, which includes a main body, an X-ray generator and an X-ray detector connected to the main body and facing each other, for radiating and detecting X-rays toward an imaging position therebetween, a support unit for supporting a subject to be imaged, and a support portion for connecting the main body to the support unit and for positioning the subject to be imaged at an imaging height between the X-ray generator and the X-ray detector, wherein the support portion horizontally moves and positions the support unit at the imaging position according to an imaging mode. According to the configuration, there are effects of increasing the convenience of the examinee and user, reducing production costs, and increasing production efficiency, by freely moving the support unit supporting an examinee's body part on an arbitrary reference plane based on the examinee's height.

## Description

### CROSS REFERENCE TO RELATED APPLICATION

The present application claims priority to Korean Patent Application No. 10-2024-0079727, filed on June 19, 2024, and Korean Patent Application No. 10-2025-0068432, filed on May 26, 2025, the entire contents of which are incorporated herein for all purposes by this reference.

### BACKGROUND

### Technical Field

The present disclosure relates to an X-ray imaging apparatus capable of freely moving a support unit for supporting a body part of an examinee on an arbitrary reference plane.

### Description of the Related Art

In general, dental clinics are equipped with X-ray imaging devices for taking X-rays in order to determine the condition of teeth and alveolar bones for the purpose of treating teeth and various periodontal diseases or correcting teeth alignment.

The X-ray imaging device used in dentistry transmits a certain amount of X-rays to the head, the body part to be imaged, and obtains an X-ray image by detecting the intensity of the transmitted X-rays and converting the same into electrical signals corresponding to the intensity of the X-rays at each location.

The X-ray imaging device described above may be, for example, a panoramic imaging device for obtaining panoramic X-ray images, a cephalometric imaging device for obtaining cephalometric X-ray images, a CT imaging device for obtaining CT images, or an X-ray imaging device capable of obtaining two or more of panoramic, cephalometric, and CT images.

A conventional technique is disclosed in Korean Patent No. 10-2258319, entitled "X-ray Imaging Apparatus and Imaging Method Thereof."

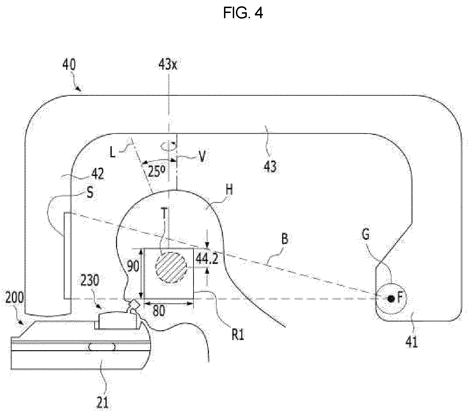

According to the above publication, as shown in FIG. 4, a gantry 40 is provided with an X-ray generator (G) and an X-ray detector (S) that are configured to face each other and rotate about a common axis of rotation. In this case, a support portion 200 for supporting a subject's head positioned between the X-ray generator and the X-ray detector during an imaging sequence involving the rotation of the X-ray generator and the x-ray detector is provided on a support frame 21 connected to the X-ray imaging device.

As discussed above, the support portion 200 having a simple frame structure in the conventional X-ray imaging apparatus is only capable of moving vertically together with the support frame 21 with respect to the main body according to the subject's height, but cannot move horizontally. Accordingly, the gantry equipped with the X-ray generator and the X-ray detector should move when the imaging position is changed according to the imaging mode, which complicates the structure and causes inconvenience to the examinee and the user.

### SUMMARY

The present disclosure is contrived to solve the above-described problem and aims to provide an X-ray imaging apparatus that increases convenience for the examinee and the user by allowing the examinee to be easily positioned at a desired imaging position and that reduces production costs and increases production efficiency because a complex moving structure for moving a support unit is unnecessary.

The present disclosure includes a main body, an X-ray generator and an X-ray detector connected to the main body and facing each other, for radiating and detecting X-rays toward an imaging position therebetween, a support unit for supporting a subject to be imaged, and a support portion for connecting the main body to the support unit and for positioning the subject to be imaged at an imaging height between the X-ray generator and the X-ray detector, wherein the support portion horizontally moves and positions the support at the imaging position according to an imaging mode.

The X-ray imaging apparatus of the present disclosure may have the following effects.

While the examinee is positioned at the imaging position by moving the support according to the imaging mode in the conventional art, the present disclosure can easily position the examinee at the desired imaging position by freely moving the support unit supporting the examinee's body part on an arbitrary reference plane based on the examinee's height, thereby increasing the convenience of the examinee and user.

In addition, because a complex moving structure for moving the support unit is unnecessary, production costs can be reduced and production efficiency can be increased.

### BRIEF DESCRIPTION OF THE DRAWINGS

FIG. 1 is a plan view showing an X-ray imaging apparatus according to a preferred exemplary embodiment of the present disclosure.
FIGS. 2 and 3 are views showing an operation of a support portion of an X-ray imaging apparatus according to a preferred exemplary embodiment of the present disclosure.
FIG. 4 is a view showing a conventional X-ray imaging apparatus.

### DETAILED DESCRIPTION OF THE DISCLOSURE

Hereinafter, a preferred exemplary embodiment according to the present disclosure will be described in detail with reference to the accompanying drawings. Prior to this, terms or words used in the present specification and claims should not be interpreted as being limited to ordinary or dictionary meanings, and should be interpreted as meanings and concepts conforming to the technical idea of the present disclosure on the basis of the principle that the inventor can appropriately define the concept of the term in order to explain his or her invention in the best way.

Accordingly, because the exemplary embodiments described in the present specification and the configuration shown in the drawings may be only the most preferred exemplary embodiment of the present disclosure and may not represent all of the technical ideas of the present disclosure, it should be understood that there are various equivalents and modifications capable of replacing them at the time of filing this application.

Like a conventional X-ray imaging apparatus described in the related art, an X-ray imaging apparatus according to a preferred exemplary embodiment of the present disclosure may include a main body 10, an X-ray generator (not shown) and an X-ray detector (not shown), which are connected to the main body 10 and facing each other, for radiating and detecting X-rays to an imaging position therebetween, a support unit 170 for supporting a subject to be imaged, such that the head part to be imaged or more precisely the chin can be placed thereon, a support portion 100 for connecting the main body 10 to the support unit 170 and for positioning the subject to be imaged at an imaging height between the X-ray generator and the X-ray detector, and a driving portion (not shown), such as a gantry, which is connected to the main body 10 and rotates the X-ray generator and the X-ray detector in opposite directions with respect to the main body, with the subject positioned therebetween.

In the conventional X-ray imaging apparatus, the support portion 100 for supporting the subject to be imaged may be fixedly installed with respect to the main body 10.

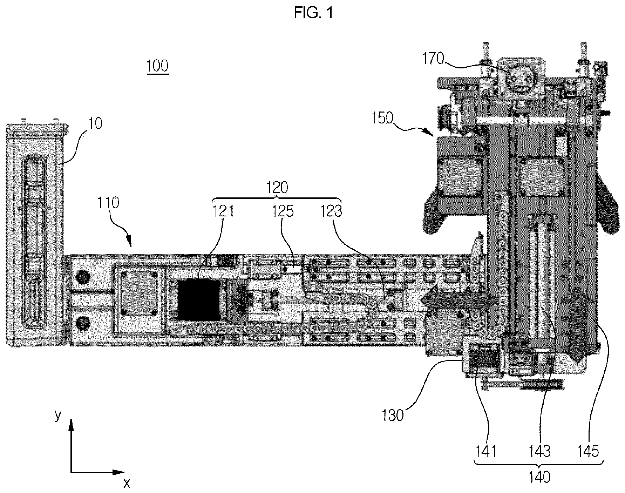

In contrast, the support unit 170 supporting the subject to be imaged in the X-ray imaging apparatus according to a preferred exemplary embodiment of the present disclosure, as shown in FIG. 1, may have a structure for freely moving along a horizontal reference plane at an arbitrary height according to the imaging position. In this case, the height of the reference plane can be determined depending on the height of the subject, and the like. That is, the support unit 170 may freely move on an arbitrary reference plane based on the imaging position.

In this way, as a preferred exemplary embodiment for moving the support unit 170, as shown in FIG. 1, the support portion 100 may include a first arm 110, a connection frame 130, and a second arm 150.

The first arm 110 may be connected to the main body 10 such as a column, and may be a frame having a long shape in one direction.

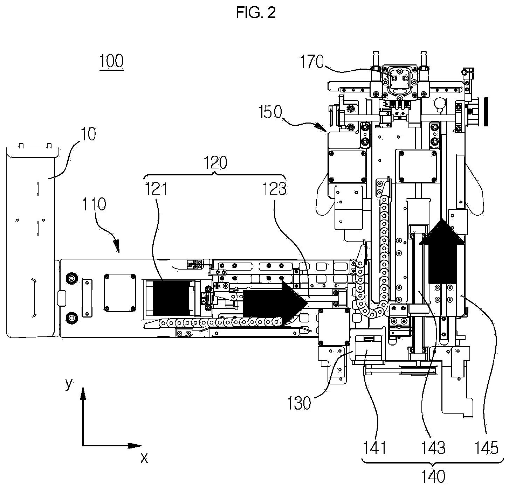

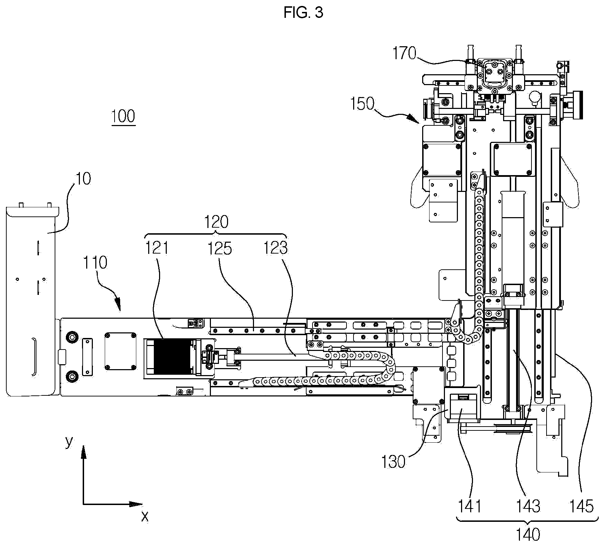

The connection frame 130 may be provided to be movable on the first arm 110 in a longitudinal direction of the first arm 110 by a first motion means 120. In the drawings of the present specification, the connection frame 130 may be provided to be movable in the x-axis direction as shown in FIGS. 2 and 3. The first motion means 120 for moving the connection frame 130 may include a first motor 121 and a first lead screw 123 as a first linear motion converting means. The first linear motion converting means 123 may be connected to a rotation shaft of the first motor 121, and the connection frame 130 may be nut-coupled to the first linear motion converting means 123, such that the rotational motion of the first motor 121 is converted into the linear motion of the connection frame 130 by the first linear motion converting means 123. In order to guide the linear motion of the connection frame 130, a first linear guide 125 may be further included as a first linear motion guiding means. The first linear motion guiding means 125 may be disposed along the longitudinal direction of the first arm 110 to guide the linear motion of the connection frame 130 with respect to the first arm 110.

The second arm 150 may be provided to be linearly movable on the connection frame 130 along a direction intersecting the first arm 110 by a second motion means 140, and a support unit 170 such as a handle frame may be connected to an end thereof. In the drawings of the present specification, the second arm 150 may be provided to be movable in a y-axis direction, the direction perpendicular to the first arm 110 as shown in FIGS. 2 and 3. The second motion means 140 for moving the second arm 150 may include a second motor 141 and a second lead screw 143 as a second linear motion converting means. The second linear motion converting means 143 may be connected to the rotation shaft of the second motor 141, and as in the present exemplary embodiment, the second linear motion converting means 143 may not be directly connected to the rotation shaft of the second motor 141 but may be connected by a belt, a pulley, or the like. By coupling the second arm 150 to the second linear motion converting means 143, the rotational motion of the second motor 141 may be converted into the linear motion of the second arm 150 by the second linear motion converting means 143. In order to guide the linear motion of the second arm 150, a second linear guide 145 may be further included as a second linear motion guiding means. The second linear motion guiding means 145 may be disposed along the longitudinal direction of the second arm 150 to guide the linear motion of the second arm 150 with respect to the connection frame 130.

With the above configuration, the position of the support unit 170 can be freely moved on the upper surface (x-y plane) of the first arm 110 through the linear motion of the connection frame 130 and the linear motion of the second arm 150, as shown in FIGS. 2 and 3.

For example, FIGS. 2 and 3 may show the position of the support unit 170 for each imaging mode, wherein in a first imaging mode of FIG. 2, the support portion 100 moves horizontally the support unit 170 along an arbitrary reference plane and places the same at a first position, and in a second imaging mode of FIG. 3 the support portion 100 moves horizontally the support unit 170 along an arbitrary reference plane and places the same at a second position different from the first position.

Herein, the first and second imaging modes may be two or more plural modes including CT, Panoramic, and Cephalo, and the CT may include a full field-of-view (FOV) CT for capturing the maximum area that the device can image, or a small field-of-view CT for capturing only a specific region within the maximum area.

In addition, the position information of the support unit 170 for each imaging mode may be stored in advance in the apparatus, such that when a user selects the imaging mode, the support portion 100 can automatically move the support unit 170 to the imaging position of the corresponding imaging mode, and, alternatively or additionally, when the user directly inputs the position information, the support portion 100 can move the support unit 170 to the corresponding position.

The X-ray imaging apparatus of the present disclosure may have the following effects.

While the examinee is positioned at the imaging position by moving the support unit according to the imaging mode in the conventional art, the present disclosure can easily position the examinee at the desired imaging position by freely moving the support unit 170 supporting the examinee's body part on an arbitrary reference plane based on the examinee's height, thereby increasing the convenience of the examinee and user.

In addition, because a complex movement structure for moving the support unit is unnecessary, production costs can be reduced and production efficiency can be increased.

Although the present disclosure has been described with reference to limited exemplary embodiments and drawings as described above, the present disclosure may not be limited thereto, and it is obvious that a person skilled in the art to which the present disclosure pertains can make various modifications and variations within the scope equivalent to the technical idea of the present disclosure and the appended claims.

## Claims

1. An X-ray imaging apparatus, the apparatus comprising:
a main body(10);
an X-ray generator connected to the main body(10) and radiating X-rays toward an imaging position;
an X-ray detector connected to the main body(10) and facing the X-ray generator and detecting X-rays passed through the imaging position;
a support unit(170) supporting a subject to be imaged; and
a support portion(100) for connecting the main body(10) to the support unit(170) and for positioning the subject at an imaging height between the X-ray generator and the X-ray detector,
wherein the imaging position varies according to an imaging mode,
**characterized in that** the support portion(100) horizontally moves and positions the support unit(170) at the imaging position according to an imaging mode.

2. The apparatus of claim 1, wherein the support portion(100) comprises:
a first arm(110) connected to the main body;
a connection frame(130) movable along a longitudinal direction of the first arm(110); and
a second arm(150) intersectingly arranged with respect to the first arm(110) to be connected to the support unit(170) and to be movable along a longitudinal direction with respect to the connection frame(130).

3. The apparatus of claim 2, further comprising: a first motion means(120) for linearly moving the connection frame(130) along the longitudinal direction with respect to the first arm(110).

4. The apparatus of claim 3, wherein the first motion means(120) comprises:
a first motor(121) provided on the first arm(110); and
a first linear motion converting means(123) disposed along the longitudinal direction of the first arm(110) and connected to the connection frame(130), for linearly moving the connection frame(130) in response to a rotational motion of the first motor(121).

5. The apparatus of claim 3, further comprising:
a first linear motion guiding means(125) disposed along the longitudinal direction of the first arm(110) for guiding a linear motion of the first arm(110).

6. The apparatus of claim 2, further comprising:
a second motion means(140) for linearly moving the second arm(150) along the longitudinal direction with respect to the connection frame(130).

7. The apparatus of claim 6, wherein the second motion means(140) comprising:
a second motor(141) provided on the connection frame(130); and
a second linear motion converting means(143) for converting a rotational motion of the second motor(141) into a linear motion of the second arm(150).

8. The apparatus of claim 7, further comprising:
a second linear motion guiding means(145) disposed along a longitudinal direction of the second arm(150) for guiding a linear motion of the second arm(150).
